# EUROPEAN PATENT SPECIFICATION

(11) **EP 1 103 275 B1**
(45) Date of publication and mention of the grant of the patent: **10.09.2003**
(21) Application number: 01104396.5
(22) Date of filing: 03.06.1996
(51) Int. Cl.: A61L 15/46, A61L 15/20

(54) **Inhibition of exoprotein in absorbent article**
Inhibierung von Exoproteinen in absorbiereden Produkten
Inhibition d'exoprotéines dans un article absorbant

(30) Priority: 07.06.1995 US 487950; 07.06.1995 US 487876; 07.06.1995 US 487875
(43) Date of publication of application: 30.05.2001
(62) Divisional of application: 96921285.1
(73) Proprietor: Kimberly-Clark Worldwide, Inc., Neenah, Wisconsin 54956 (US)
(72) Inventor: Syverson, Rae Ellen, Fond du Lac, Wisconsin 54935 (US)
(74) Representative: Diehl, Hermann, Dr.

(56) References cited:
- EP-A- 0 395 099
- EP-A- 0 402 266
- EP-A- 0 483 812
- WO-A-86/05388
- US-A- 3 629 454
- US-A- 4 405 323

## Description

The present invention relates to the inhibition of exoprotein in an absorbent article, such as vaginal tampons and sanitary napkins. More particularly, the invention relates to the incorporation of amine compounds either alone or in combination with one or more ether compounds, and/or amide compounds into such absorbent articles and these compounds effects on Gram positive bacteria.

### BACKGROUND OF THE INVENTION

Disposable absorbent devices for the absorption of human exudates are widely used. These disposable devices typically have a compressed mass of absorbent formed into the desired shape, which is typically dictated by the intended consumer use. In the area of a menstrual tampon, the device is intended to be inserted in a body cavity for absorption of the body fluids generally discharged during a woman's menstrual period.

There exists in the female body a complex process which maintains the vagina and physiologically related areas in a healthy state. In a female between the age of menarche and menopause, the normal vagina provides an ecosystem for a variety of microorganisms. Bacteria are the predominant type of microorganism present in the vagina; most women harbor about 10⁹ bacteria per gram of vaginal exudate. The bacterial flora of the vagina is comprised of both aerobic and anaerobic bacteria. The more commonly isolated bacteria are Lactobacillus species, corynebacteria, Gardnerella vaginalis, Staphylococcus species, Peptococcus species, aerobic and anaerobic Streptococcal species, and Bacteroides species. Other microorganisms that have been isolated from the vagina on occasion include yeast (Candida albicans), protozoa (Trichomonas vaginalis), mycoplasma (Mycoplasma hominis), chlamydia (Chlamydia trachomatis). and viruses (Herpes simplex). These latter organisms are generally associated with vaginitis or venereal disease, although they may be present in low numbers without causing symptoms.

Physiological, social and idiosyncratic factors affect the quantity and species of bacteria present in the vagina. Physiological factors include age, days of the menstrual cycle, and pregnancy. For example, vaginal flora present in the vagina throughout the menstrual cycle can include lactobacilli, corynebacterium, ureaplasma, and mycoplasma. Social and idiosyncratic factors include method of birth control, sexual practices, systemic disease (e.g. diabetes), and medication.

Bacterial proteins and metabolic products produced in the vagina can affect other microorganisms and the human host. For example, the vagina between menstrual periods is mildly acidic having a pH ranging from about 3.8 to about 4.5. This pH range is generally considered the most favorable condition for the maintenance of normal flora. At that pH, the vagina normally harbors the numerous species of microorganisms in a balanced ecology, playing a beneficial role in providing protection and resistance to infection and makes the vagina inhospitable to some species of bacteria such as Staphylococcus aureus (S. aureus). The low pH is a consequence of the growth of lactobacilli and their production of acidic products. Microorganisms in the vagina can also produce antimicrobial compounds such as hydrogen peroxide and bactericides directed at other bacterial species. One example is the lactocins, bacteriocin-like products of lactobacilli directed against other species of lactobacilli.

Some microbial products may affect the human host. For example, S. aureus can produce and excrete into its environment a variety of exoproteins including enterotoxins, Toxic Shock Syndrome Toxin-1 (TSST-1), and enzymes such as proteases and lipase.

S. aureus is found in the vagina of approximately 16% of healthy women of menstrual age. Approximately 25% of the S. aureus isolated from the vagina are capable of producing TSST-1. TSST-1 and some of the staphylococcal enterotoxins have been identified as causing Toxic Shock Syndrome (TSS) in humans.

Symptoms of TSS generally include fever, diarrhea, vomiting and a rash followed by a rapid drop in blood pressure. Systemic vital organ failure occurs in approximately 6% of those who contact the disease. S. aureus does not initiate TSS as a result of the invasion of the microorganism into the vaginal cavity. Instead as S. aureus grows and multiplies, it can produce Toxic Shock Syndrome Toxin 1 (TSST-1; synonyms: pyrogenic exotoxin C and enterotoxin F). Only after entering the bloodstream does the TSST-1 toxin act systemically and produce the symptoms attributed to Toxic Shock Syndrome.

Menstrual fluid has a pH of approximately 7.3. During menses, the pH of the vagina moves toward neutral and can become slightly alkaline. This change permits microorganisms whose growth is inhibited by an acidic environment the opportunity to proliferate. For example, S. aureus is more frequently isolated from vaginal swabs during menstruation than from swabs collected between menstrual periods.

There have been numerous attempts to reduce or eliminate pathogenic microorganisms and menstrually occurring TSS by incorporating into a tampon pledget one or more biostatic, biocidial, and/or detoxifying compounds. For example, L-ascorbic acid has been applied to a menstrual tampon to detoxify toxin found in the vagina of the human female during menstruation.

Incorporating glyceryl triacetate into a tampon pledget has been suggested. Glyceryl triacetate is readily broken down into glycerol and acetic add by the enzymatic action of esterase. Esterase is present in the vaginal epithelium and in menstrual fluid. The enzymatic action of the esterase is in turn controlled by the pH of the environment, being more active when the pH is on the alkaline side. Since the pH of the vagina moves toward the alkaline side during menstruation, the enzymatic activity of the esterase automatically increases and attacks the glyceryl triacetate. This releases acetic acid rapidly, which has the potential to reduce the pH and enzymatic activity of the esterase. However, menstrual fluid is well buffered and the acetic acid is ineffective at lowering the pH of the menstrual fluid.

Others have incorporated monoesters and diesters of polyhydric aliphatic alcohols and a fatty acid containing from 8 to 18 carbon atoms. For example, glycerol monolaurate (GML) has been used to inhibit the production of S. aureus enterotoxins and TSST-1. However, as noted above, esterase is abundantly present in the vaginal epithelium and menstrual fluid. This esterase, in combination with esterase and lipase produced by bacteria can enzymatically degrade the esters into non-effective compounds (*see e.g.* EP-A-483812).

Until now, persons skilled in the art have not appreciated the affects of lipase and esterase on ester compounds. Thus, one or more ester compounds may have to be added to the absorbent article, such as a tampon pledget, in sufficiently high concentrations to detrimentally effect the normal flora present in the vaginal area. When'the natural condition is altered, overgrowth by pathogen(s) may take place resulting in a condition known as vaginitis.

Accordingly, there exists a need for an absorbent product that has incorporated therein a compound that will: effectively inhibit the production of exoproteins, such as TSST-1, from Gram positive bacterium; will be substantially unaffected by the enzymes lipase and esterase; and will not substantially alter the natural flora found in the vaginal area.

### SUMMARY OF THE INVENTION

Briefly, the present invention is based on the discovery that when an effective amount of one or more amine compounds or amine salts, either alone or in combination with ether and/or amide compositions are incorporated into an absorbent article, such as a catamenial tampon, the production of exoprotein from Gram positive bacterium is substantially inhibited.

Ether compounds which may be used in combination with the amine compounds of the invention are represented by the general formula:

R₁-O-R₂

wherein R₁ is a straight or branched chain alkyl group having from 8 to 18 carbon atoms and R₂ is selected from an alcohol, a polyalkoxylated sulfate salt, or a polyalkoxylated sulfosuccinate salt. It has been observed that when these ether compound are incorporated into an absorbent article, such as a catamenial tampon, the production of exoprotein in Gram positive bacterium is substantially inhibited.

Amine compounds of the invention are represented by the general formula: 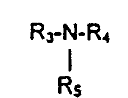 wherein R₃, is an alkyl group having from 8 to 18 carbon atoms; and R₄ and R₅ can be the same or different and are independently selected from hydrogen and alkyl group(s) having from 1 to 18 carbon atoms. The alkyl groups of R₄ and R₅ can include one or more substitutional moieties selected from hydroxyl, carboxyl and carboxyl salts and imidazoline. R₃ and R₄ can also form an unsaturated heterocyclic ring that contains a nitrogen that connects via a double bond to the alpha carbon of the R₅ moiety to form a substituted imidazoline. It is also within the scope of this invention for the amine compound to include an amine salt. It has been observed that amine compounds and salts thereof are effective in substantially inhibiting the production of exoprotein from Gram positive bacteria. Amide compositions which may be used in combination with the amine compounds of the invention are represented by the general formula: 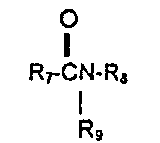 wherein R₇, inclusive of the carbonyl carbon, is an alkyl group having 8 to 18 carbon atoms and R₈ and R₉ can be the same or different. R₅ and R₉ are selected from hydrogen and an alkyl group having 1 to 12 carbon atoms. The alkyl group of R₈ and R₉ may contain one or more substituent groups selected from ester, ether, amine, hydroxyl, carboxyl, carboxyl salts, sulfonate and sulfonate salts.

It is a general object of the invention to provide an absorbent article which inhibits the production of exoproteins from Gram positive bacterium. A more specific object of the invention is to provide a catamenial tampon incorporating one or more amine compounds, either alone or in combination with ether and/or amide compositions which act to substantially inhibit the production of TSST-1 and Enterotoxin B by S. aureus.

Another object of the invention is to provide a catamenial tampon that has incorporated therewith one or more amine compounds, either alone or in combination with ether and/or amide compositions that will substantially inhibit the production of exoproteins from Gram positive bacterium without significantly imbalancing the natural flora present in the vaginal tract.

Another object of the invention is to provide a method for inhibiting the production of exoprotein produced by Gram positive bacteria in an absorbent article.

Other objects and advantages of the invention, and modifications thereof, will become apparent to persons skilled in the art without departure from the inventive concepts defined in the appended claims.

### DETAILED DESCRIPTION OF THE PREFERRED EMBODIMENTS

This invention will be described in detail in connection with a catamenial tampon but would be understood by persons skilled in the art to be applicable to other disposable absorbent articles, such as: sanitary napkins, panty liners, adult incontinent undergarments, diapers, medical bandages and tampons such as those intended for medical, dental, surgical, and/or nasal use wherein inhibition of exoproteins from Gram positive bacteria would be beneficial.

Vaginal tampons suitable for use in this invention are usually made of absorbent fibers, including natural and synthetic fibers, compressed into a unitary body of a size which may easily be inserted into the vaginal cavity. They are normally made in an elongated cylindrical form in order that they may have a sufficiently large body of material to provide the required absorbing capacity, but can be made in a variety of shapes. The tampon may or may not be compressed, although compressed types are now generally preferred. The tampon can be made of various fiber blends including both absorbent and nonabsorbent fibers, which may or may not have a suitable cover or wrapper.

It has been found that certain ether compounds can substantially inhibit the production of exoprotein of Gram positive bacterium, more specifically, the production of TSST-1 and Enterotoxin B from S. aureus bacterium. The ether compounds which may be used in combination with the amine compounds of the present invention have the general formula:

R₁-O-R₂

wherein R₁ is a straight or branched alkyl group having a chain of 8 to 18 carbon atoms and R₂ is selected from an alcohol, a polyalkoxylated sulfate salt or a polyalkoxylated sulfosuccinate salt.

The alkyl, or the R₁ moiety of the ether compounds useful herein, can be derived from saturated and unsaturated fatty acid compounds. Suitable compounds include, C₈-C₁₈ fatty adds, and preferably, the fatty adds include, without limitation, caprylic, capric, lauric, myristic, palmitic and stearic acid whose carbon chain lengths are 8, 10, 12, 14, 16 and 18, respectively. Highly preferred materials include capric, lauric, and myristic.

Preferred unsaturated fatty acids are those having one or two cis-type double bonds and mixtures of these materials. Suitable materials include myrystoleic, palmitoleic, linolenic and mixtures thereof.

Desirably, the R₂ moiety is an aliphatic alcohol which can be ethoxylated or propoxylated for use in the compositions of the present invention. Suitable aliphatic alcohols include glycerol, sucrose, glucose, sorbitol, sorbitan and derivatives thereof. Preferred ethoxylated and propoxylated alcohols include glycols such as ethylene glycol, propylene glycol, polyethylene glycol and polypropylene glycol.

The aliphatic alcohols can be ethoxylated or propoxylated by conventional ethoxylating or propoxylating compounds and techniques. The compounds are preferably selected from the group consisting of ethylene oxide, propylene oxide, and mixtures thereof, and similar ringed compounds which provide a material which is effective. Most preferably, the ethoxylation compound is selected from the group consisting of ethylene oxide, propylene oxide and mixtures thereof.

The R₂ moiety can further include polyalkoxylated sulfate and polyalkoxylated sulfosuccinate salts. The salts can have one or more cations. Preferably, the cations are sodium, potassium or both.

Preferred ether compounds include laureth-3, laureth-4, laureth-5, PPG-5 lauryl ether, 1-0-dodecyl-rac-glycerol, sodium laureth sulfate, potassium laureth sulfate, disodium laureth (3) sulfosuccinate, dipotassium laureth (3) sulfosuccinate and polyethylene oxide (2) sorbitol ether.

In accordance with this invention, the tampon in addition to the amine compounds according to the invention may contain an effective amount of the inhibiting ether compound to substantially inhibit the formation of TSST-1 when the tampon is exposed to S. aureus bacteria. Effective amounts have been found to be at least 0.005 millimoles of ether compound per gram of absorbent. Preferably, the ether compound ranges from 0.005 millimoles per gram of absorbent to 2 millimoles per gram of absorbent and more preferably 0.005 millimoles per gram of absorbent to 0.2 millimoles per gram of absorbent. Although "compound" is used in the singular, one skilled in the art would understand that it includes the plural. That is, the absorbent article can include more than one ether compound.

According to the present invention amine compounds substantially inhibit the production of exoprotein of Gram positive bacteria, and more specifically, the production of TSST-1 and Enterotoxin B from S. aureus bacterium. Specifically, the amines and their salts have the general formula: 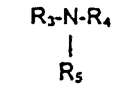 wherein R₃ is an alkyl group having from 8 to 18 carbon atoms; and R₄ and R₅ can be the same or different and are selected from hydrogen and alkyl group(s) having from 1 to 18 carbon atoms. The R₄ and R₅ alkyl groups can include one or more substitutional moieties selected from hydroxyl, carboxyl and carboxyl salts and imidazoline. The amine compounds of the invention are effective in substantially inhibiting the production of exoprotein from Gram positive bacteria.

It is critical to the invention that the R₃ moiety be an alkyl group having from 8 to 18 carbon atoms. Desirably, the alkyl group is derived from fatty acid compounds which include, without limitation, caprylic, capric, lauric, myristic, palmitic and stearic acid whose carbon chain lengths are 8, 10, 12, 14, 16 and 18, respectively. Highly preferred materials include capric, lauric, and myristic. Preferred unsaturated fatty acids are those having one or two cis-type double bonds and mixtures of these materials. Suitable materials include myrystoleic, palmitoleic, linolenic and mixtures thereof.

The R₄ and R₅ alkyl groups can further include one or more substitutional moieties selected from hydroxyl, carboxyl, carboxyl salts, and R₃ and R₄ can form an unsaturated heterocyclic ring that contains a nitrogen that connects via a double bond to the alpha carbon of the R₅ moiety to form a substituted imidazoline. The carboxyl salts can have one or more cations selected from sodium, potassium or both. The R₃-R₅ alkyl groups can be straight or branched and can be saturated or unsaturated.

In another embodiment, the amine compound can be a salt. The salt is represented by the general formula: 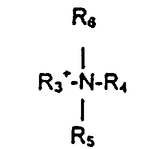 wherein R₃ is an anionic moiety associated with the amine and is derived from an alkyl group having from 8 to 18 carbon atoms. Desirably, R₃ is a polyalkyloxylated alkyl sulfate. The R₆ moiety is the same as that described above for the R₄ and R₅ moieties. The R₃-R₆ alkyl groups can be straight or branched and can be saturated or unsaturated. A preferred compound illustrative of an amine salt is TEA laureth sulfate.

Preferred amine compounds of the present invention which may be incorporated into the absorbent article or onto the cover thereof include: TEA laureth sulfate, lauramine, lauramino propionic acid, sodium lauriminodipropionic acid, lauryl hydroxyethyl imidazoline and mixtures thereof.

In accordance with the invention, the tampon contains an effective amount of the inhibiting amine compound to substantially inhibit the formation of TSST-1 when the tampon is exposed to S. aureus bacteria. Effective amounts have been found to be at least 1 x (10⁻⁵) millimoles of the amine compound per gram of absorbent. Preferably, the amine compound ranges from 0.005 millimoles per gram of absorbent to 2 millimoles per gram of absorbent and more preferably 0.005 millimoles per gram of absorbent to 0.2 millimoles per gram of absorbent. Although "compound" is used in the singular, one skilled in the art would understand that it includes the plural. That is, the absorbent article can include more than one amine compound.

In another embodiment of the invention, the amine compounds of the invention may be combined with amide compounds having the general formula: 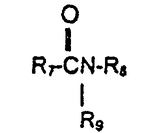 wherein R₇, inclusive of the carbonyl carbon, is an alkyl group having 8 to 18 carbon atoms and R₈ and R₉ can be the same or different. R₈ and R₉ are selected from hydrogen and an alkyl group having 1 to 12 carbon atoms which may contain one or more substituent groups selected from ester, ether, amine, hydroxyl, carboxyl, carboxyl salts, sulfonate and sulfonate salts.

The alkyl moiety, which includes the carbonyl carbon, can be derived from saturated and unsaturated fatty acid compounds. Suitable compounds include, C₈-C₁₈ fatty acids, and preferably, the fatty acids include, without limitation, caprylic, capric, lauric, myristic, palmitic and stearic acid whose carbon chain lengths are 8, 10, 12, 14, 16 and 18, respectively. Highly preferred materials include capric, lauric, and myristic.

Preferred unsaturated fatty acids are those having one or two cis-type double bonds and mixtures of these materials. Suitable materials include myrystoleic, palmitoleic, linolenic and mixtures thereof.

The R₈ and R₉ moieties can be the same or different and each being selected from hydrogen and an alkyl group having a carbon chain having from 1 to 12 carbon atoms. The R₇-R₉ alkyl groups can be straight or branched and can be saturated or unsaturated. When R₈ and/or R₉ are an alkyl moiety having a carbon chain of at least 2 carbons, the alkyl group can include one or more substituent groups selected from ester, ether, amine, hydroxyl, carboxyl, carboxyl salts, sulfonate and sulfonate salts. The salts can have one or more cations selected from sodium, potassium or both.

Preferred amide compounds include sodium lauroyl sarcosinate, lauramide MEA, lauramide DEA, lauramidopropyl dimethylamine, disodium lauramido MEA sulfosuccinate and disodium lauroamphodiacetate.

In accordance with the invention, the tampon may contain an effective amount of amide compound in addition to the inhibiting amine compound according to the invention to substantially inhibit the formation of TSST-1 when the tampon is exposed to S. aureus bacteria. Effective amounts have been found to be at least 5 x (10⁻⁴) millimoles of the amide compound per gram of absorbent. Preferably, the amide compound ranges from 0.005 millimoles per gram of absorbent to 2 millimoles per gram of absorbent. More preferably the amide compound ranges from 0.005 millimoles per gram of absorbent to 0.2 millimoles per gram of absorbent. Although "compound" is used in the singular, one skilled in the art would understand that it includes the plural. That is, the absorbent article can include more than one amide compound.

The compositions of the present invention can be prepared and applied in any suitable form, but are preferably prepared in forms including, without limitation aqueous solutions, lotions, balms, gels, salves, ointments, boluses, suppositories, and the like.

The compositions may be applied to the absorbent article using conventional methods for applying an inhibitory agent to the desired absorbent article. For example, unitary tampons without separate wrappers, may be dipped directly into a liquid bath having the agent and then can be air dried, if necessary to remove any volatile solvents. For compressed tampons, impregnating of any of its elements is best done before compressing. The compositions when incorporated on and/or into the tampon materials may be fugitive, loosely adhered, bound, or any combination thereof. As used herein the term "fugitive" means that the composition is capable of migrating through the tampon materials.

It is not necessary to impregnate the entire absorbent body of the tampon with the inhibitory agent. Optimum results both economically and functionally, can be obtained by concentrating the material on or near the outer surface where it will be most effective during use.

The substantially inhibitory composition may additionally employ one or more conventional pharmaceutically-acceptable and compatible carrier materials useful for the desired application. The carrier can be capable of co-dissolving or suspending the materials used-in-the composition. Carrier materials suitable for use in the instant composition, therefore, include those well-known for use in the cosmetic and medical arts as a basis for ointments, lotions, creams, salves, aerosols, suppositories, gels and the like.

The inhibitory compositions of the present invention may additionally employ adjunct components conventionally found in pharmaceutical compositions in their art-established fashion and at their art-established levels. For example, the compositions may contain additional compatible pharmaceutically active materials for combination therapy, such as supplementary antimicrobials, anti-parasitic agents, antipruritics, astringents, local anesthetics, or anti-inflammatory agents.

The present invention may be readily understood by considering the following examples illustrative of specific embodiments. The Examples are given to serve as a guide In carrying out the invention and are not to be construed as a limitation or limitations of the invention. It will be understood that various changes or modifications may be made, as will be apparent to those skilled in the art, without departing from the scope of the claims annexed hereto.

### EXAMPLES 1-3 (Examples 1 and 3 are reference examples)

The efficacy of the test compounds on TSST-1 production was determined by placing the desired concentration, expressed in millimoleslmilliliter (millimolar hereinafter mM) of the active compound in 10 milliliters of a Growth Medium of each test compound in a Coming 50 ml conical polystyrene tube. The polystyrene tube is available from Scientific Products Division, Baxter Diagnostics Incorporated, 1430 Waukegan Road, McGaw Park, IL 60085-6787.

The Growth Medium was prepared as follows: Brain heart infusion broth (BHI), available from Becton Dickinson Microbiology Systems, Cockeysville, MD 21030, was dissolved and sterilized according to the manufacturer's instructions. Ninety milliliters of BHI broth was supplemented with 10 ml fetal bovine serum (FBS), available from Sigma Chemical Company, P.O. Box 14508, St. Louis, MO 63178-9916. One milliliter of a 0.02 molar sterile solution of the hexahydrate of magnesium chloride, available from Sigma Chemical Company, was added to the BHI-FBS mixture. One milliliter of a 0.027 molar sterile solution of L-glutamine available from Sigma Chemical Company was also added to the BHI-FBS mixture.

If the test compound was not water soluble or water miscible, it was first dissolved at 50 times the desired concentration in 10 ml isopropanol, then diluted to the desired final concentration in 10 ml of the Growth Medium. Tubes of Growth Medium with an equivalent amount of isopropanol, but no test compound, were prepared as controls.

In preparation for inoculation of the tubes of Growth Medium containing the test compound, an inoculating broth was prepared as follows. S. aureus, (MN8) was streaked onto a sheep blood agar plate and incubated at 37°C. The test organism in this Example was obtained from Dr. Pat Schlievert, Department of Microbiology of the University of Minnesota Medical School, Minneapolis, MN. After 24 hours of incubation three to five individual colonies were picked with a sterile inoculating loop and used to inoculate 10 ml of the Growth Medium. The tube of inoculated Growth Medium was capped with a S/P® diSPo® plug available from Scientific Products Division, Baxter Diagnostics, Incorporated and incubated at 37°C in atmospheric air having 5% by volume CO₂. After 24 hours of incubation, the culture was removed from the incubator and mixed well on a S/P brand vortex mixer. A second tube containing 10 ml of the Growth Medium was inoculated with 0.5 ml of the above 24 hour culture and re-incubated at 37°C in atmospheric air having 5% by volume CO₂. After 24 hours of incubation the culture was removed from the incubator and mixed well on a S/P brand vortex mixer. Each tube of Growth Medium containing a test compound and growth control tubes with or without isopropanol were inoculated with 0.1 ml of the prepared inoculating broth. The initial colony forming units (CFU) per ml of Growth Medium were approximately 1 x 10⁷. The tubes were capped with S/P® diSPo® plugs and incubated at 37°C in atmospheric air having 5% by volume CO₂. After 24 hours of incubation the tubes were removed from the incubator and the culture fluid was assayed for the number of colony forming units of S. aureus and prepared for analysis of TSST-1 per method described below.

The number of colony forming units per ml after incubation was determined by standard plate count procedure. The culture fluid broth was centrifuged and the supernatant subsequently filter sterilized through an Acrodisc® syringe filter unit available from Scientific Products Division, Baxter Diagnostics, Inc. The resulting fluid was frozen at -80°C until assayed.

The amount of TSST-1 per milliliter was determined by a non-competitive, sandwich enzyme-linked immunoabsorbent assay (ELISA). Samples of the culture fluid and the TSST-1 reference standard were assayed in triplicate. The method employed was as follows: Four reagents, rabbit polyclonal anti-TSST-1 IgG (#LTI-101), rabbit polyclonal anti-TSST-1 IgG conjugated to horseradish peroxidase (#LTC-101), TSST-1 (#TT-606), and normal rabbit serum certified anti-TSST-1 free (#NRS-10) were purchased from Toxin Technology, Incorporated, 7165 Curtiss Avenue, Sarasota, Florida, 34231. Sixty-two microliters of polyclonal rabbit anti-TSST-1 IgG (#LTI-101) was appropriately diluted so that a 1:100 dilution gave an absorbency of 0.4 at 650 nanometers. This was added to 6.5 ml of 0.5 molar carbonate buffer, pH 9.6, and 100 microliters of this solution was pipetted into the inner wells of polystyrene microtiter plates #439454, obtained from Nunc-Denmark. The plates were covered and incubated overnight at 37°C. Unbound antitoxin was removed by three washes with phosphate buffered saline (pH 7.2) (0.011 molar NaH₂PO₄ and 0.9% [wt/vol] NaCI both available from Sigma Chemical Company) containing 0.5% [vol/vol] Tween 20 (PBS-Tween), also available from Sigma Chemical Company. The plates were treated with 100 microliters of a 1% [wt/vol] solution of bovine serum albumin (BSA), available from Sigma Chemical Company, covered, and incubated at 37°C for one hour. Unbound BSA was removed by 6 washes with PBS-Tween. TSST-1 reference standard, serially diluted from 1 - 10 ng/ml in PBS-Tween, test samples treated with normal rabbit serum 10% [vol/vol] final concentration and reagent controls were pipetted in 100 microliter volumes to their respective wells. This was followed by incubation for two hours at 37°C and three washes to remove unbound toxin. The rabbit polyclonal anti-TSST-1 IgG conjugated to horseradish peroxidase and diluted according to manufacturer's instructions was added (100 microliter volumes) to each microtiter well. The plates were covered and incubated at 37°C for one hour.

Following incubation the plates were washed 6 times in PBS-Tween. Following this, the wells were treated with a solution consisting of 0.075 molar sodium citrate (pH 4.0), 0.6 millimolar 2,2'-Azino-bis-(3-ethylbenzthiazoline-6-sulfonic acid) diammonium salt and 0.009% [vol/vol] hydrogenperoxide, all available from Sigma Chemical Company. The intensity of the color reaction in each well was evaluated over time using a BioTek Model EL340 Microplate reader (OD 405 nm) and Kineticalc® software available from Biotek Instruments, Inc. TSST-1 concentrations in test samples were predicted from the reference toxin regression equations derived during each assay procedure.

The efficacy of the amine compounds of the present invention, as well as of ether and amide compounds as reference examples in inhibiting the production of TSST-1 is shown below in Tables I-III, respectively.

**TABLE I**

| | | | |
|---|---|---|---|
| (Reference) | | | |

| Compound | mM Test Compound | CFU/ml | ELISA: TSST-1 ng/ml |
|---|---|---|---|
| Growth control | None | 3.3 X 10⁹ | 381.8 |
| 1-0-dodecyl-rac-glycerol | 10.67 | 1.2 X 10⁹ | 19.8 |
| Laureth-3 | 9.03 | 2.4 X 10⁸ | 3.7 |
| Laureth-4 | 10.20 | 2.4 X 10⁸ | 2.3 |
| Sodium laureth sulfate | 10.65 | 2.9 X 10³ | ND |
| PPG-5 Laureth-5 | 7.35 | 2.0 X 10⁸ | 1.6 |
| Disodium laureth | | | |
| sulfosuccinate | 9.84 | 3.4 X 10⁸ | 2.3 |
| ND = None detected | | | |

The above list of compounds (Commercial Name), their percent active compound, and vendor are as follows:
1-0-dodecyl-rac-glycerol, Sigma Chemical Company, 100%, P.O. Box 14508, St. Louis, MO 63178-9916.
Laureth-3, (Trycol 5966), 97%, Henkle Corporation, Emery Group, 4900 Este Avenue, Cincinnati, Ohio, 45232.
Laureth-4, (Trycol 5882), 100%, Henkle Corporation, 300 Brookside Avenue, Ambler, Pennsylvania 19002.
Sodium laureth sulfate, (Standapol ES-2), 25%, Henkle Corporation. PPG-5 Laureth-5, (Aethoxal B), 100%, Henkle Corporation.
Disodium laureth sulfosuccinate, (Standapul SH124-3), 39%, Henkle Corporation.

The data in Table I show that S. aureus MN8, when compared to the control, produced significantly less TSST-1 in the presence of the ether compounds. The ether compounds reduced the amount of exotoxin production ranging from about 95 percent to greater than 99.6 percent. However, although the amount of toxin produced was significantly reduced, in most cases the ether compounds did not substantially reduce the number of S. aureus cells.

**TABLE II**

| Compound | mM Test Compound | CFU/ml | ELISA: TSST-1 ng/ml |
|---|---|---|---|
| Growth Control | None | 2.5 x 10⁹ | 153.2 |
| Laurimino propionic acid | 10.67 | No Growth | <1.1 |
| | 0.43 | Not Determined* | 24.2 |
| Sodium lauriminodipropionic | 10.67 | 1.0 x 10³ | <1.1 |
| acid | 2.15 | Not Determined | 2.9 |
| Lauryl hydroxyethyl | 10.67 | No Growth | <1.1 |
| imidazoline | 0.43 | Not Determined | 85.9 |
| TEA laureth sulfate | 10.67 | 6.2 x 10² | <1.1 |
| | 2.15 | None Detected | 4.7 |
| Lauramine | 10.67 | No Growth | <1.1 |
| | 2.15 | Not Determined | 77.0 |

| | | | |
|---|---|---|---|
| * S. aureus grew in the culture broth, the amount of growth was not determined. | | | |

The above list of compounds (Commercial Name), their percent active compound, and vendor are as follows:
Laurimino propionic acid, (Mackam 151L), 40%, McIntyre Group, LTD, 1000 Governors Highway, University Park, IL. 60466.
Sodium lauriminodipropionic add, (Deriphat 160-C.), 30%, Henkle/Cospha, Cospha Group, 300 Brookside Ave., Ambler, PA 19002.
Lauryl hydroxyethyl imidazoline, (Schercozoline L), Scher Chemicals, Inc., Industrial West, P.O. Box 4317, Clifton, NJ 07012.
TEA laureth sulfate, (Sulfochem TLES), 39%, Chemron, P O Box 2299.
Paso-Robles, CA 93447.
Lauramine,(Dodecylamine), 99+, Aldrich, 1001 West Saint Paul Ave, Milwaukee, WI 53233.

In accordance with the present invention, the data in Table It show that S. aureus MN8, when compared to the control, produced significantly less TSST-1 in the presence of the amine compounds. The amine compounds reduced the amount of exotoxin production ranging from about 86 percent to greater than 99.4 percent.

**TABLE III**

| (Reference) | | | |
|---|---|---|---|
| Compound | mM Test Compound | CFU/ml | ELISA: TSST-1 ng/ml |
| Growth Control | None | 3.1 x 10⁹ | 381.8 |
| | | | |
| Lauramide MEA | 10.67 | 1.5 x 10⁹ | 51.6 |
| | | | |
| Sodium lauroyl sarcosinate | 10.70 | 1.4 x 10³ | <1.1 |
| | | | |
| Disodium lauroamphodiacetate | 10.74 | 3.5 x 10⁸ | 2.9 |
| | | | |
| Disodium lauramido MEA sulfosuccinate | 10.71 | 9.1 x 10⁸ | 1.2 |

The above list of compounds (Commercial Name), their percent active compound, and vendor are as follows:
Lauramide MEA, (Comperlan LNN), 98.5%, Henkle Corporation, 300 Brookside Avenue, Ambler, Pennsylvania 19002.
Sodium lauroyl sarcosinate, (Hamposyl L-30), 30%, Hampshire Chemical Co., 55 Hayden Ave., Lexington, MA 02173.
Disodium lauroamphodiacetate, (Mackam 2-L), 50% McIntyre Group, 1000 Governors Highway, University Park, IL. 60466.
Disodium lauramido MEA sulfosuccinate, (Mackanate LM-40), 40%, McIntyre Group, 1000 Governors Highway, University Park, IL. 60466.

The data in Table III show that S. aureus MN8, when compared to the control, produced significantly less TSST-1 in the presence of the amide compounds. The amide compounds reduced the amount of exotoxin production ranging from about 86 percent to greater than 99.6 percent.

### EXAMPLES 4-6 (Examples 4 and 6 are reference examples)

The efficacy of the test compounds in reducing the production of a second exoprotein of S. aureus was determined using S. aureus HOCH, a known producer of Enterotoxin B The test organism in this Example was obtained from Dr. Pat Schlievert, Department of Microbiology of the University of Minnesota Medical School, Minneapolis, MN. The experimental procedure for assessing the efficacy of the test compounds effect on growth of S. aureus HOCH and for production of a culture filtrate was the same as set forth in Example A above.

The amount of S. aureus enterotoxin B per milliliter was determined by Western Blot assay. Samples of the culture fluid and the Staphylococcal Enterotoxin B (SEB) reference standard were assayed in triplicate. The method employed was similar to that described in "A Rapid, Sensitive Method for Detection of Alkaline Phosphatase-Conjugated Anti-Antibody on Western Blots," M.S. Blake, K.H. Johnston, G.J. Russell-Jones and E. C. Gotschlich, Analytical Biochemistry, 136:175-179, 1984. Enterotoxin B was separated from other proteins in the test samples by sodium dodecyl sulfate-polyacrylamide gel (SDS-PAGE) electrophoresis. The upper stacking gel used 3% acrylamide gel, the lower separating gel contained 14% acrylamide gel. The upper gel was prepared with a comb containing twenty lanes spanning 15.5 centimeters of the top of the stacking gel. A low molecular weight SDS-PAGE standard, BioRad #161-0305 available from Bio-Rad Laboratories having offices at 2000 Alfred Nobel Drive, Hercules, CA 94547, SEB, #BT-202 from Toxin Technology, Incorporated, and culture extracts, as prepared above, were each mixed 1:1 with a loading buffer. The loading buffer contained 30% [vol/vol] glycerol, 15% [vol/vol] mercaptoethanol, 7% [wt/vol] sodium dodecyl sulfate, 0.0036% [wt/vol] bromphenol blue, and 0.76% [wt/vol] Trizma base with a pH of 6.8. The mixtures were boiled for 5 minutes, then twenty-five (25) microliters of each mixture was placed in a lane. The SDS-PAGE gel was electrophoresed (60 to 80 volts) for 90 minutes or until the dye front was through the stacking gel and for an additional 2.5 hours (160-170 volts) with an electrophoresis buffer of 0.61% [wt/vol] Tris base, 2.85% [wt/vol] glycine, 0.1% [wt/vol] SDS, pH 7.85. Proteins were transferred to a nitrocellulose transfer membrane, available from Schleicher and Schull, Inc., Keene, N.H. 03431, #BA 85, overnight approximately 15 hours at 200 milliamps in a Bio-Rad Trans-Blot® cell. The transfer buffer was composed of 0.3% [wt/vol] Tris base, 1.4% [wt/vol] glycine, 20% [vol/vol] methanol, pH 7.6.

The nitrocellulose membrane was treated for 45 minutes at 37°C with 3% [wt/vol] gelatin in 0.02 molar Tris buffer, 0.5 molar NaCI, at pH 7.5 (TBS) to block non-specific reactions, then washed at 37°C with TBS-0.05% [vol/vol] Tween 20 (TBS-Tween) for 45 minutes. The membrane was then submerged for 1.5 hours at 37°C in 50 ml TBS-Tween containing 0.05 ml rabbit polyclonal anti-SEB IgG, #LBI-202 available from Toxin Technology, Incorporated.

The membrane was washed two times in TBS-Tween, then submerged a second time for 1.5 hours at 37°C in a 50 ml solution of TBS-Tween with 25 microliters of goat anti-rabbit IgG conjugated to alkaline phosphatase. The membrane was washed twice in TBS-Tween and twice in TBS. The blot was developed with a reaction solution consisting of 2 mg 5-bromo-4-chloroindolyl phosphatase, 100 microliters of N,N dimethyl formamide, 18 ml 0.15 molar barbitol buffer, pH 9.2, 2 mg nitroblue tetrazolium, and 40 microliters of a 2 molar solution of MgCl₂ ·6H₂O, all available from Sigma. The reaction was stopped with a cold water wash. The amount of SEB produced in the presence of the test compound was estimated by a comparison with the staining intensity produced by a serial dilution of SEB.

The efficacy of the amine compounds of the present invention, as well as of ether and amide compounds as reference examples in inhibiting the production of Enterotoxin B is shown below in Tables IV-VI, respectively.

**TABLE IV**

| (Reference) | | | |
|---|---|---|---|
| Compound | mM Test Compound | CFU/ml | Western Blot: SEB mg/ml |
| Growth control | None | 1.0 X 10⁹ | 0.8 |
| 1-0-dodecyl-rac-glycerol | 10.67 | 7.0 X 10⁸ | 0.8 |
| Laureth-3 | 9.03 | 8.3 X 10⁸ | ND |
| Laureth-4 | 10.20 | 7.5 X 10⁸ | ND |
| Sodium laureth sulfate | 2.13 | 1.2 X 10⁷ | ND |
| PPG-5 Laureth-5 | 7.35 | 6.4 X 10⁸ | ND |
| Disodium laureth | | | |
| sulfosuccinate | 9.84 | 2.1 X 10⁷ | ND |
| ND = None detected, <0.16 mg/ml | | | |

The data in Table IV show that S. aureus:hOCH, when compared to the control, produced less SEB in the presence of the ether compounds. However, although the amount of toxin produced was substantially reduced, the ether compounds did not significantly reduce the number of S. aureus cells.

**TABLE V**

| Compound | mM Test Compound | CFU/ml | Western Blot: SEB mg/ml |
|---|---|---|---|
| Growth control | None | 7.0 x 10⁹ | 0.8 |
| | | | |
| Laurimino | 10.67 | No growth | None detected |
| propionic acid | 0.43 | Not determined* | None detected |
| | | | |
| Sodium | 10.67 | 1.3 x 10³ | None detected |
| lauriminodipropionic | | | |
| acid | 2.15 | Not determined | None detected |
| | | | |
| Lauryl | | | |
| hydroxyethyl | 10.67 | No growth | None detected |
| imidazoline | 0.43 | Not determined | None detected |
| | | | |
| TEA laureth | 10.67 | 5.6 x 10² | None detected |
| sulfate | 2.15 | Not determined | None detected |
| | | | |
| Lauramine | 10.67 | No growth | None Detected |
| | 2.15 | Not determined | 0.2 |

| | | | |
|---|---|---|---|
| * S. aureus grew in the culture broth, the amount of growth was not determined. | | | |

In accordance with the present invention, the data in Table II show that S. aureus HOCH produced significantly less SEB in the presence of the amine compounds. Compared to the control, the amine compounds reduced the amount of exotoxin production below the detectable range of 0.16 milligrams/milliliter.

**TABLE IV**

| (Reference) | | | |
|---|---|---|---|
| Compound | mM Test | CFU/ml | Western Blot: SEB mg/ml |
| Growth Control | None | 1.5 x 10⁹ | 0.8 |
| | | | |
| Lauramide MEA | 10.67 | 3.7 x 10⁸ | None Detected |
| | | | |
| Sodium lauroyl sarcosinate | 10.70* | 1.7 x 10³ | None Detected |
| | | | |
| Disodium lauroamphodiacetate | 10.74 | 1.2 x 10⁸ | None Detected |
| | | | |
| Disodium lauramido MEA sulfosuccinate | 10.71 | 1.2 x 10⁸ | None Detected |

| | | | |
|---|---|---|---|
| * No SEB detected at 0.43 millimole concentration of sodium lauroyl sarcosinate, no plate count was performed. None Detected = <0.16 mg/ml. | | | |

The data in Table VI show that S. aureus KOCH, when compared to the control, produced significantly less SEB in the presence of the amide compounds. However, although the amount of toxin produced was reduced below detectable level, the concentration of the amide compound can be adjusted so that there was little reduction in the number of S. aureus cells. The amide compounds reduced the amount of exotoxin production below the detectable range of 0.16 milligrams/milliliter.

Another aspect of the invention is for a method of inhibiting the production of exoprotein from Gram positive bacteria in an absorbent product. The method includes the steps of contacting the absorbent article, such as a tampon, with one or more of the above described inhibitory compositions then placing the absorbent article for use so that the production of exoprotein from Gram positive bacteria is inhibited. The absorbent article can have one or more of the inhibitory compositions absorbed into or coated onto the fibers or cover material. Desirably, the production of TSST-1 and Enterotoxin B are inhibited.

While the invention has been described in conjunction with a specific embodiment, it is to be understood that many alternatives, modifications and variations will be apparent to those skilled in the art in light of foregoing description. Accordingly, this invention is intended to embrace all such alternatives, modifications and variations which fall within the scope of the appended claims.

## Claims

1. An absorbent article comprising an effective amount of one or more nitrogen containing compounds having the general formula: 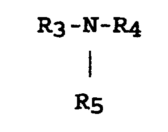 wherein R₃ is an alkyl group having from 8 to 18 carbon atoms; and R₄ and R₅ can be the same or different and are selected from hydrogen and an alkyl group having from 1 to 18 carbon atoms and which can have one or more substitutional moieties selected from hydroxyl, carboxyl and carboxyl salts, and imidazoline or wherein R₃ and R₄ can form an unsaturated heterocyclic ring that contains a nitrogen that connects via a double bond to the alpha carbon of the R₅ moiety to form a substituted imidazoline, wherein said compound is effective in substantially inhibiting the production of exoprotein from Gram positive bacteria.

2. The absorbent article of claim 1 wherein said R₃-R₅ alkyl group is straight.

3. The absorbent article of claim 1 wherein said R₃-R₅ alkyl group is branched.

4. The absorbent article of claim 1 wherein R₃ is selected from an alkyl group derived from caprylic, capric, lauric, myristic, palmitic or stearic acid.

5. The absorbent article of claim 1 wherein said carboxyl salt has a cationic moiety selected from sodium, potassium or both.

6. The absorbent article of claim 1 wherein said nitrogen containing compound is selected from lauramine, lauramino propionic acid, sodium lauriminodipropionic acid, lauryl hydroxyethyl imidazoline and mixtures thereof.

7. The absorbent article of claim 1 wherein said compound is present in an amount greater than 1 x (10⁻⁵) millimoles per gram of absorbent.

8. The absorbent article of claim 1 wherein said compound is present in an amount ranging from 0.005 millimoles per gram of absorbent to 2 millimoles per gram of absorbent.

9. An absorbent article comprising an effective amount of one or more nitrogen containing salts having the general formula: 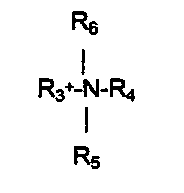 wherein R₃+ is an anionic moiety derived from an alkyl group having from 8 to 18 carbon atoms; and R₄-R₆ can be the same or different and are selected from hydrogen and an alkyl group having from 1 to 18 carbon atoms and which can have one or more substitutional moieties selected from hydroxyl, carboxyl and carboxyl salts, and imidazoline wherein said compound is effective in substantially inhibiting the production of exoprotein from Gram positive bacteria.

10. The absorbent article of claim 9 wherein said nitrogen containing salt is TEA laureth sulfate.

11. The absorbent article of any of the preceding claims wherein said one or more nitrogen containing compounds are used in combination with an effective amount of one or more nitrogen containing compounds having the general formula: 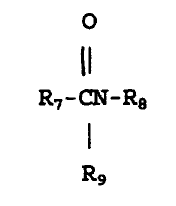 wherein R₇ is an alkyl group having 8 to 18 carbon atoms, inclusive of the carbonyl carbon, and R₈ and R₉ can be the same or different, R₈ and R₉ are selected from hydrogen, an alkyl group having 1 to 12 carbon atoms and may contain one or more substituent groups selected from ester, ether, amine, hydroxyl, carboxyl, carboxyl salts, sulfonate, sulfonate salts and combinations thereof, wherein said compound is effective in substantially inhibiting the production of exoprotein from Gram positive bacteria and/or an effective amount of one or more ether compounds having the general formula:
R₁-O-R₂
wherein R₁ is a straight or branched alkyl group having a chain of 8 to 18 carbon atoms and R₂ is selected from an alcohol, a polyalkoxylated sulfate salt and a polyalkoxylated sulfosuccinate salt, wherein said compound is effective in substantially inhibiting the production of exoprotein from Gram positive bacteria.

12. A method of inhibiting the production of exoprotein from Gram positive bacteria in an absorbent product comprising the steps of contacting said absorbent product with an effective amount of one or more nitrogen containing compounds and exposing said absorbent product to at least one Gram positive bacteria, said nitrogen containing compound having the general formula: 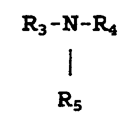 wherein R₃ is an alkyl group having from 8 to 18 carbon atoms; and R₄ and R₅ can be the same or different and are selected from hydrogen and an alkyl group having from 1 to 18 carbon atoms and which can have one or more substitutional moieties selected from hydroxyl, carboxyl and carboxyl salts, and imidazoline.

13. The method of claim 12 wherein said compound is present in an amount greater than 5 x (10⁻⁴) millimoles per gram of absorbent of said tampon.

14. The method of claim 12 wherein said Gram positive bacteria is TSST-1 producing Staphylococcus aureus bacteria.

15. The method of claim 12 wherein said Gram positive bacteria is Enterotoxin B producing Staphylococcus aureus bacteria.

16. The method of claim 12 wherein said nitrogen containing compound is selected from TEA laureth sulfate, lauramine, lauramino propionic acid, sodium lauriminodipropionic acid, lauryl pyridinium chloride, lauryl hydroxyethyl imidazoline and mixtures thereof.

17. The method of any of claims 12 to 16 wherein said absorbent product is contacted additionally with an effective amount of one or more nitrogen containing compounds having the general formula: 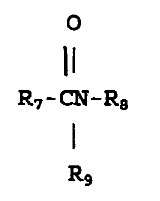 wherein R₇ is an alkyl group having 8 to 18 carbon atoms, inclusive of the carbonyl carbon, and R₈ and R₉ can be the same or different, R₈ and R₉ are selected from hydrogen, an alkyl group having 1 to 12 carbon atoms and may contain one or more substituent groups selected from ester, ether, amine, hydroxyl, carboxyl, carboxyl salts, sulfonate, sulfonate salts and combinations thereof, wherein said compound is effective in substantially inhibiting the production of exoprotein from Gram positive bacteria and/or an effective amount of one or more ether compounds having the general formula:
R₁ - O - R₂
wherein R₁ is a straight or branched alkyl group having a chain of 8 to 18 carbon atoms and R₂ is selected from an alcohol, a polyalkoxylated sulfate salt and a polyalkoxylated sulfosuccinate salt, wherein said compound is effective in substantially inhibiting the production of exoprotein from Gram positive bacteria.

## Patentansprüche

1. Absorbierender Gegenstand umfassend eine wirksame Menge einer oder mehrerer Stickstoff beinhaltenden Verbindungen mit der allgemeinen Formel: 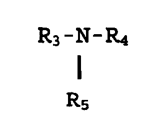 wobei R₃ eine Alkylgruppe mit 8 bis 18 Kohlenstoffatomen ist; und R₄ und R₅ gleich oder verschieden sein können und ausgewählt sind aus Wasserstoff und einer Alkylgruppe mit 1 bis 18 Kohlenstoffatomen und die nur eine oder mehrere Substituentengruppen aufweisen, ausgewählt aus Hydroxyl, Carboxyl und Carboxylsalzen und Imidazolin oder wobei R₃ und R₄ einen ungesättigten heterocyclischen Ring bilden können, welcher einen Stickstoff beinhaltet, der über eine Doppelbindung mit dem alpha-Kohlenstoff der R₅-Gruppierung verbunden ist und so ein substituiertes Imidazolin bildet,
wobei die Verbindung wirksam ist, die Produktion von Exoprotein von grampositiven Bakterien wesentlich zu inhibieren.

2. Absorbierender Gegenstand nach Anspruch 1, wobei die R₄-R₅.Alkylgruppen geradkettig sind.

3. Absorbierender Gegenstand nach Anspruch 1, wobei die R₃-R₅-Alkylgruppen verzweigt sind.

4. Absorbierender Gegenstand nach Anspruch 1, wobei R₃ ausgewählt ist aus einer Alkylgruppe, die abgeleitet ist von Capryl-, Caprin-, Laurin-, Myristin-, Palmitin- oder Stearinsäure.

5. Absorbierender Gegenstand nach Anspruch 1, wobei das Carboxylsalz eine kationische Gruppierung aufweist, die ausgewählt ist aus Natrium, Kalium, oder beiden.

6. Absorbierender Gegenstand nach Anspruch 1, wobei die stickstoffhaltige Verbindung ausgewählt ist aus Lauramin, Lauraminopropionsäure, Natriumlauraminodipropionsäure, Laurylhydroxyethylimidazolin und Mischungen davon.

7. Absorbierender Gegenstand nach Anspruch 1, wobei die Verbindung in einer Menge größer als 1 x (10⁻⁵) Millimol pro Gramm Absorbens vorhanden ist.

8. Absorbierender Gegenstand nach Anspruch 1, wobei die Verbindung in einer Menge von 0,005 Millimol pro Gramm Absorbens bis 2 Millimol pro Gramm Absorbens vorhanden ist.

9. Absorbierender Gegenstand umfassend eine wirksame Menge eines oder mehrerer stickstoffhaltiger Salze mit der allgemeinen Formel: 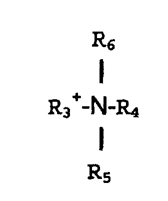 wobei R₃⁺ eine anionische Gruppierung ist, die aus einer Alkylgruppe mit 8 bis 18 Kohlenstoffatomen die abgeleitet ist; und R₄-R₆ gleich oder verschieden sein können und ausgewählt sind aus Wasserstoff und einer Alkylgruppe mit 1 bis 18 Kohlenstoffatomen und welche eine oder mehrere Substituentengruppen aufweisen können, ausgewählt aus Hydroxyl, Carboxyl und Carboxylsalzen, und Imidazolin, wobei die Verbindung wirksam ist, die Produktion von Exoprotein von grampositiven Bakterien wesentlich zu inhibieren.

10. Absorbierender Gegenstand nach Anspruch 9, wobei das stickstoffhaltige Salz TEA Laurethsulfat ist.

11. Absorbierender Gegenstand nach einem der vorhergehenden Ansprüche, wobei die ein oder mehreren stickstoffhaltigen Verbindungen in Kombination mit einer wirksamen Menge einer oder mehrerer Stickstoff beinhaltenden Verbindungen mit der allgemeinen Formel: 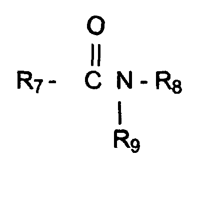 verwendet werden, wobei R₇ eine Alkylgruppe mit 8 bis 18 Kohlenstoffatomen ist, einschließlich des Carbonylkohlenstoffs ist, und R₈ und R₉ gleich oder verschieden sein können, wobei R₈ und R₉ ausgewählt sind aus Wasserstoff, einer Alkylgruppe mit 1 bis 12 Kohlenstoffatomen, und eine oder mehrere Substituentengruppen ausgewählt aus Ester, Ether, Amin, Hydroxyl, Carboxyl, Carboxylsalzen, Sulfonat, Sulfonatsalzen und Kombinationen davon, wobei die Verbindung wirksam ist, die Produktion von Exoprotein von grampositiven Bakterien wesentlich zu inhibieren und/oder eine wirksame Menge einer oder mehrerer Etherverbindungen mit der allgemeinen Formel:
R₁ - O - R₂
wobei R₁ eine geradkettige oder verzweigte Alkylgruppe mit einer Kette von 8 bis 18 Kohlenstoffatomen ist und R₂ ausgewählt ist aus einem Alkohol, einem polyalkoxylierten Sulfatsalz und einem polyalkoxylierten Sulfosuccinatsalz, wobei die Verbindung wirksam ist, die Produktion von Exoprotein von grampositiven Bakterien wesentlich zu inhibieren.

12. Verfahren zur wesentlichen Inhibierung der Produktion von Exoprotein von grampositiven Bakterien in einem absorbierenden Gegenstand, umfassend die Schritte des inKontaktbringens des absorbierenden Produkts mit einer wirksamen Menge einer oder mehrerer Stickstoff beinhaltenden Verbindungen und Aussetzen des absorbierenden Gegenstands wenigstens einem grampositiven Bakterium, wobei die Stickstoff beinhaltende Verbindung die allgemeine Formel: 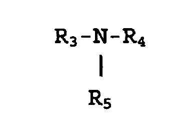 hat, wobei R₃ eine Alkylgruppe mit 8 bis 18 Kohlenstoffatomen ist; und R₄ und R₅ gleich oder verschieden sein können und ausgewählt sind aus Wasserstoff und einer Alkylgruppe mit 1 bis 18 Kohlenstoffatomen, und welche eine oder mehrere Substituentengruppen ausgewählt aus Hydroxyl, Carboxyl, Carboxylsalzen, und Imidazolin aufweisen kann.

13. Verfahren nach Anspruch 12, wobei die Verbindung in einer Menge größer als 5 x (10⁻⁴) Millimol pro Gramm Absorbens des Tampons vorhanden ist.

14. Verfahren nach Anspruch 12, wobei die grampositiven Bakterien TSST-1 produzierende Staphylococcus-aureus Bakterien sind.

15. Verfahren nach Anspruch 12, wobei die grampositiven Bakterien Enterotoxin B produzierende Staphylococcus-aureus Bakterien sind.

16. Verfahren nach Anspruch 12, wobei die Stickstoff beinhaltende Verbindung ausgewählt ist aus der Gruppe aus TEA Laurethsulfat, Lauramin, Lauraminpropionsäure, Natriumlauraminodipropionsäure, Laurylpyridiniumchlorid, Laurylhydroxyethylimidazolin und Mischungen davon.

17. Verfahren nach Anspruch 12 bis 16, wobei der absorbierende Gegenstand zusätzlich in Kontakt gebracht wird mit einer wirksamen Menge einer oder mehrerer Stickstoff beinhaltenden Verbindungen mit der allgemeinen Formel: 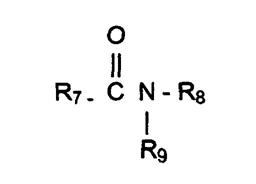 wobei R₇ eine Alkylgruppe mit 8 bis 18 Kohlenstoffatomen ist, einschließlich des Carbonyl-Kohlenstoffs, und R₈ und R₉ gleich oder verschieden sein können, wobei R₈ und R₉ ausgewählt sind aus Wasserstoff, einer Alkylgruppe mit 1 bis 12 Kohlenstoffatomen und eine oder mehrere Substituentengruppen beinhalten können, die ausgewählt sind aus Ester, Ether, Amin, Hydroxyl, Carboxyl, Carboxylsalzen, Sulfonaten, Sulfonatsalzen, und Mischungen davon, wobei die Verbindung wirksam ist, die Produktion von Exoprotein von grampositiven Bakterien wesentlich zu inhibieren und/oder eine wirksamen Menge einer oder mehrerer Etherverbindungen mit der allgemeinen Formel:
R₁ - O - R₂
wobei R₁ eine geradkettige oder verzweigte Alkylgruppe mit einer Kette von 8 bis 18 Kohlenstoffatomen ist und R₂ ausgewählt ist aus einem Alkohol, einem polyalkoxylierten Sulfatsalz und einem polyalkoxylierten Sulfosuccinatsalz, wobei die Verbindung wirksam ist, die Produktion von Exoprotein von grampositiven Bakterien wesentlich zu inhibieren.

## Revendications

1. Article absorbant comprenant une quantité efficace d'un ou plusieurs composés contenant de l'azote ayant la formule générale : 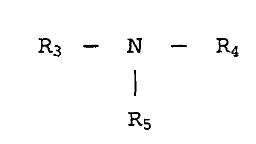 dans laquelle R₃ est un groupe alkyle ayant de 8 à 18 atomes de carbone ; et R₄ et R₅ peuvent être identiques ou différents et sont sélectionnés parmi l'hydrogène et un groupe alkyle ayant de 1 à 18 atomes de carbone et qui peuvent avoir un ou plusieurs motifs substituants sélectionnés parmi hydroxyle, carboxyle, les sels carboxyliques et l'imidazoline, ou dans laquelle R₃ et R₄ peuvent former un noyau hétérocyclique insaturé qui contient un atome d'azote se connectant via une double liaison au carbone en alpha sur le motif R₅ pour former une imidazoline substituée, ledit composé étant efficace pour sensiblement inhiber la production d'exoprotéine par des bactéries Gram positives.

2. Article absorbant selon la revendication 1, dans lequel ledit groupe alkyle en R₃-R₅ est à chaîne droite.

3. Article absorbant selon la revendication 1, dans lequel ledit groupe alkyle en R₃-R₅ est à chaîne ramifiée.

4. Article absorbant selon la revendication 1, dans lequel R₃ est sélectionné parmi les groupes alkyle dérivant des acides caprylique, caprique, laurique, myristique, palmitique ou stéarique.

5. Article absorbant selon la revendication 1, dans lequel ledit sel carboxylique comporte un motif cationique sélectionné entre le sodium, le potassium ou les deux.

6. Article absorbant selon la revendication 1, dans lequel ledit composé contenant de l'azote est sélectionné entre la lauramine, l'acide lauramino-propionique, l'acide laurimino-dipropionique sodique, la lauryl-hydroxyéthyl imidazoline et leurs mélanges.

7. Article absorbant selon la revendication 1, dans lequel ledit composé est présent en une quantité supérieure à 1 x (10⁻⁵) millimole par gramme d'absorbant.

8. Article absorbant selon la revendication 1, dans lequel ledit composé est présent en une quantité comprise entre 0,005 millimole par gramme d'absorbant et 2 millimoles par gramme d'absorbant.

9. Article absorbant comprenant une quantité efficace d'un ou plusieurs sels contenant de l'azote ayant la formule générale : 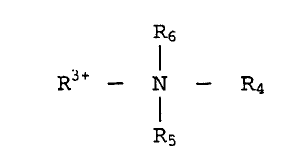 dans laquelle R₃⁺ est un motif anionique dérivé d'un groupe alkyle ayant de 8 à 18 atomes de carbone ; et R₄-R₆ peuvent être identiques ou différents et sont choisis parmi l'hydrogène et un groupe alkyle ayant de 1 à 18 atomes de carbone et qui peut avoir un ou plusieurs motifs substituants sélectionnés parmi hydroxyle, carboxyle, sels carboxyliques et imidazoline, ledit composé étant efficace à inhiber sensiblement la production d'exoprotéine par des bactéries Gram positives.

10. Article absorbant selon la revendication 9, dans lequel ledit sel contenant de l'azote est le TEA laureth sulfate.

11. Article absorbant selon l'une quelconque des revendications précédentes, dans lequel lesdits un ou plusieurs composés contenant de l'azote sont utilisés en combinaison avec une quantité efficace d'un ou plusieurs composés contenant de l'azote ayant la formule générale : 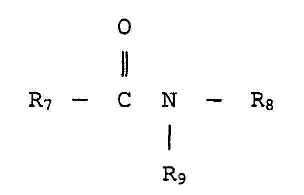 dans laquelle R₇ est un groupe alkyle ayant de 8 à 18 atomes de carbone, y compris le carbone de la fonction carbonyle, et R₈ et R₉ peuvent être identiques ou différents, R₈ et R₉ sont sélectionnés parmi l'hydrogène, un groupe alkyle ayant de 1 à 12 atomes de carbone et qui peut contenir un ou plusieurs groupes substituants sélectionnés parmi ester, éther, amine, hydroxyle, carboxyle, sels carboxyliques, sulfonate, sels sulfonates et leurs combinaisons, ledit composé étant efficace à inhiber sensiblement la production d'exoprotéine par des bactéries Gram positives et/ou une quantité efficace d'un ou plusieurs composés éthers ayant la formule générale :
R₁ - O - R₂
dans laquelle R₁ est un groupe alkyle linéaire ou ramifié ayant une chaîne de 8 à 18 atomes de carbone et R₂ est sélectionné parmi un alcool, un sel de sulfate polyalkoxylé et un sel de sulfosuccinate polyalkoxylé, ledit composé étant efficace à inhiber sensiblement la production d'exoprotéine par des bactéries Gram positives.

12. Procédé d'inhibition de la production d'exoprotéine par des bactéries Gram positives dans un produit absorbant, comprenant les étapes de mise en contact dudit produit absorbant avec une quantité efficace d'un ou plusieurs composés contenant de l'azote et l'exposition dudit produit absorbant à au moins une bactérie Gram positive, ledit composé contenant de l'azote ayant la formule générale : 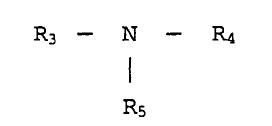 dans laquelle R₃ est un groupe alkyle ayant de 8 à 18 atomes de carbone ; et R₄ et R₅ peuvent être identiques ou différents et sont sélectionnés parmi l'hydrogène et un groupe alkyle ayant de 1 à 18 atomes de carbone et qui peut avoir un ou plusieurs motifs substituants sélectionnés parmi hydroxyle, carboxyle, sels carboxyliques et imidazoline.

13. Procédé selon la revendication 12, dans lequel ledit composé est présent en une quantité supérieure à 5 x 10⁻⁴ millimole par gramme d'absorbant dudit tampon.

14. Procédé selon la revendication 12, dans lequel ladite bactérie Gram positive est la bactérie Staphylococcus aureus produisant du TSST-1.

15. Procédé selon la revendication 12, dans lequel ladite bactérie Gram positive est la bactérie Staphylococcus aureus produisant de l'entérotoxine B.

16. Procédé selon la revendication 12, dans lequel ledit composé contenant de l'azote est sélectionné parmi le TEA laureth sulfate, la lauramine, l'acide lauramino-propionique, l'acide laurimino-dipropionique sodique, le chlorure de lauryl-pyridinium, la lauryl-hydroxyéthyle imidazoline et leurs mélanges.

17. Procédé selon l'une quelconque des revendications 12 à 16, dans lequel ledit produit absorbant est mis en contact en outre avec une quantité efficace d'un ou plusieurs composés contenant de l'azote ayant la formule générale : 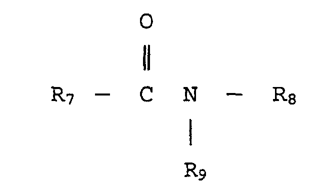 dans laquelle R₇ est un groupe alkyle ayant de 8 à 18 atomes de carbone, y compris le carbone de la fonction carbonyle, et R₈ et R₉ peuvent être identiques ou différents, R₈ et R₉ sont sélectionnés parmi l'hydrogène, un groupe alkyle ayant de 1 à 12 atomes de carbone et qui peut contenir un ou plusieurs groupes substituants sélectionnés parmi ester, éther, amine, hydroxyle, carboxyle, sels carboxyltques, sulfonate, sels sulfonates et leurs combinaisons, ledit composé étant efficace à inhiber sensiblement la production d'exoprotéine par des bactéries Gram positives et/ou une quantité efficace d'un ou plusieurs composés éthers ayant la formule générale :
R₁ - O - R₂
dans laquelle R₁ est un groupe alkyle linéaire ou ramifié ayant une chaîne de 8 à 18 atomes de carbone et R₂ est sélectionné parmi un alcool, un sel de sulfate polyalkoxylé et un sel de sulfosuccinate polyalkoxylé, ledit composé étant efficace à inhiber sensiblement la production d'exoprotéine par des bactéries Gram positives.
